# EUROPEAN PATENT APPLICATION

(11) **EP 2 698 631 A2**
(43) Date of publication of application: **19.02.2014**
(21) Application number: 12762868.3
(22) Date of filing: 09.03.2012
(51) Int. Cl.: G01N 33/52, G01N 21/00, G01N 1/36

(54) **OPTICAL SENSOR STRIP AND DIAGNOSTIC DEVICE COMPRISING SAME**

(30) Priority: 25.03.2011 KR 20110026658
(71) Applicant: Micobiomed Co., Ltd., Daejon 306-809 (KR)
(72) Inventor: PARK, Hyun Kyu, Daejeon 305-345 (KR); LEE, Joong Jin, Incheon 403-030 (KR); KU, Renata, Okcheon-gun Chungcheongbuk-do 373-881 (KR)
(74) Representative: Zaboliene, Reda
(86) International application number: PCT/KR2012/001758
(87) International publication number: WO 2012/134074

(57) **Abstract**

Disclosed are an optical-sensor strip that can reduce a measurement error and a diagnostic apparatus having the optical-sensor strip. An embodiment includes: a plate-like support that has a plurality of insertion protrusions on a surface thereof on which a sample pad containing a sample is to be mounted; a cover that is placed on the support, has a plurality of pairs of injection holes, into which the insertion protrusions are inserted, and includes a plurality of injection holes that are formed to penetrate a body of the cover; and a fixing frame with a guide portion in which the support and the cover are inserted in a combined state.

## Description

### BACKGROUND OF THE INVENTION

### Field of the Invention

The present invention relates to an optical-sensor strip and a diagnostic apparatus having the same and more particularly to an optical-sensor strip having a structure in which a support and a cover are inserted in a fixing frame in a superimposed state so that it receives a uniform pressing force and a diagnostic apparatus having the optical-sensor strip.

### Description of the Related Art

The total cholesterol in blood, plasma, or serum is known as one kind of biometric data that represents a risk index of arteriosclerosis. However, recent clinical researches have reported that there is a relationship between a concentration of low-density lipoprotein (LDL) cholesterol and arteriosclerosis. For such a reason, measurement of a concentration of the LDL cholesterol is believed to be a more useful method of determining risk of arteriosclerosis than measurement of the total cholesterol.

As a method of measuring LDL cholesterol, there is a method of using a measurement strip containing a reaction reagent. Most measurement strips have a stacked structure in which a plurality of pads that contains a reaction regent are interposed between a cover as an upper layer and a support as a lower layer. The measurement strip has a vertical flow channel, a blood filtering layer, and a color-induced membrane.

An optical-sensor strip according to a related art uses a method of inserting a membrane filter and a regular grid net into a measurement portion of a strip using an adhesive made of a macromolecular polymer material. This structure has advantages of a simple structure and an easy combining operation, but also has disadvantages such that there is a limitation on structural diversity, a large amount of blood should be dispensed, and a strip is likely to be easily deformed.

In order to eliminate the disadvantages, a plastic extrusion-molded product is used in which an upper part and a lower part are combined, using male and female columns rather than using an adhesive, and folded. Examples of a combining and folding system include a pole structure, a hook structure, or the like. This method has advantages of a reduction in an amount of blood to be dispensed and structural diversity. However, according to this method, when extrusion-molded products are folded at the center so as to be superimposed, it is difficult to adjust a degree of compression so that it is required to uniformly apply an external force to the molded products. Moreover, a degree of the contraction of the combined structure affects a reaction speed between a dispensed solution and a reaction substance at a reaction site of the extrusion-molded products, and it also affects reproducibility of the values of repetitive measurements.

Moreover, the strip according to the related art has a structure in which it is inserted in a longitudinal direction when it is inserted into a diagnostic apparatus. Accordingly, the strip comes into direct contact with an insertion unit of the diagnostic apparatus, so that the strip is occasionally bent or deformed in the thickness direction during the insertion due to interference.

### SUMMARY OF THE INVENTION

Accordingly, the present invention has been made keeping in mind the above problems occurring in the related art, and is intended to provide.

Accordingly, the present invention is directed to an optical-sensor strip having improved safety and reliability by maintaining a uniform gap between a support and a cover of the strip that substantially obviates one or more problems due to limitations and disadvantages of the related art.

The present invention is also directed to an optical-sensor strip having a structure in which a strip is inserted into a fixing frame so that it can be inserted into a diagnostic apparatus in a widthwise direction, which prevents the strip from being deformed during an insertion operation, and a diagnostic apparatus equipped with the optical-sensor strip.

The present invention is further directed to an optical-sensor strip with a cover that has a plurality of injection holes, thereby being capable of diagnosing simultaneously a plurality of samples.

In order to achieve the above object, according to one aspect of the present invention, there is provided an optical-sensor strip including: a support including a plate shape, the support having a plurality of insertion protrusions 21 formed on an upper surface thereof on which a sample pad containing a sample is mounted, a cover placed on the upper surface of the support, the cover including insertion holes to engage with the insertion protrusions, and a plurality of injection holes penetrating the cover, and a fixing frame including a guide portion in which a combined structure of the support and the cover is inserted.

The cover may be provided with injection protrusions that protrude from a surface thereof along a circumference of the injection holes.

The support may include through holes that are formed to penetrate a body of the support at positions corresponding to the injection holes, and at least two insertion protrusions 21 may be symmetrically arranged at around the through hole.

The support may include gap-forming protrusions at both end portions thereof for maintaining a gap between the cover and the support.

At a center portion of each of the gap-forming protrusion, an additional insertion protrusion may be provided.

The fixing frame may include a base plate having a handle portion at a side thereof, and the guide portion may have wall portions protruding from a surface of the base plate to extend along both sides of the combined structure of the support and the cover, and projected portions bent from end of each of the wall portions in a direction approaching each other.

The fixing frame may have a barrier wall that closes an open end of the guide portion.

A stopping recess may be formed on an end portion of a lower surface of the support and a stopping protrusion is formed on the base plate so that the stopping protrusion is temporarily received in the stopping recess when the support is fully inserted in the guide portion.

An end portion of the support may have a slopped surface.

According to another aspect, there is provided a diagnostic apparatus including the optical-sensor strip, a body having an insertion unit in which the optical-sensor strip is inserted in a widthwise direction of the optical-sensor strip, and a multi-injector including a plurality of cuvettes with end portions inserted in the injection holes, and supporting protrusions that are protruded in a longitudinal direction of the cuvettes, and ends of the supporting protrusions may be in contact with the cover so that a state in which the end portions of the cuvettes are inserted in the injection holes is remained.

An optical-sensor strip according to the invention reduces a measurement error due to a combination error, thereby improving reliability of a measured value.

Moreover, since a cover receives a uniform external force due to a fixing frame and a uniform gap is maintained between the cover and a support due to gap-forming protrusions, the reliability of the measurement value improves.

The support and the cover are inserted into the fixing frame in a superimposed state, and the fixing frame is inserted in such a state into an insertion unit of a body in a widthwise direction. Accordingly, it is possible to prevent the strip from being deformed due to interference during an inserting operation, so that a safety and reliability of the strip are improved.

Moreover, it is possible to charge samples into the strip in a state in which respective end portions of multi-cuvettes are partially engaged with injection protrusions protruding along injection holes of the cover. Accordingly, it is possible to prevent unintended samples from being charged due to a user's mistake, which improves safety and reliability of the optical-sensor strip.

### BRIEF DESCRIPTION OF THE DRAWINGS

The above and other objects, features and other advantages of the present invention will be more clearly understood from the following detailed description when taken in conjunction with the accompanying drawings, in which:
Fig. 1 is an exploded perspective view illustrating an optical-sensor strip according to an embodiment of the invention;
Figs. 2A and 2B are cross-sectional views illustrating a support and a cover shown in Fig. 1;
Fig. 3 is a cross-sectional view illustrating a state in which the support and the cover are inserted into a fixing frame; and
Fig. 4 is a plan view illustrating a diagnostic apparatus according to an embodiment of the invention.

### DETAILED DESCRIPTION OF THE INVENTION

Hereinafter, an optical-sensor strip according to an embodiment of the invention, and a diagnostic apparatus equipped with the optical-sensor strip will be described with reference to the accompanying drawings.

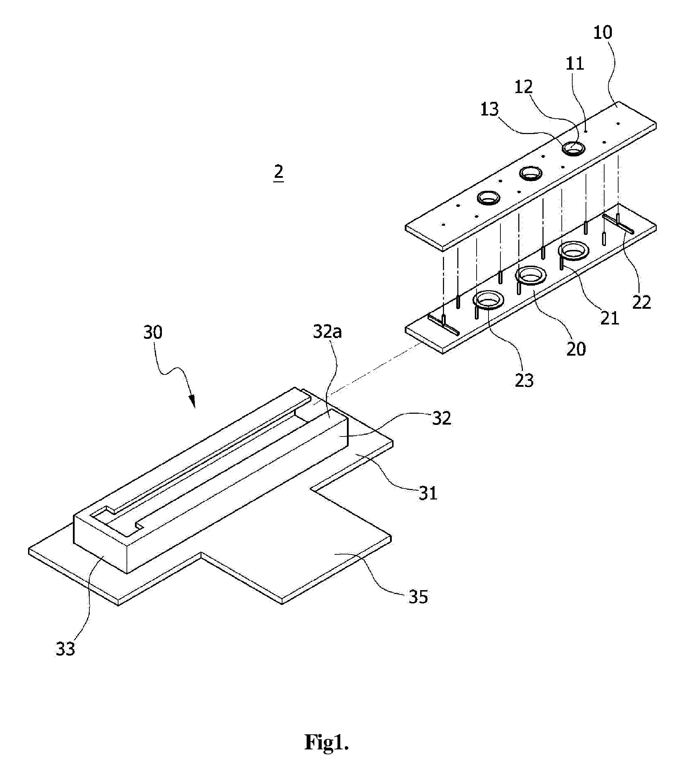

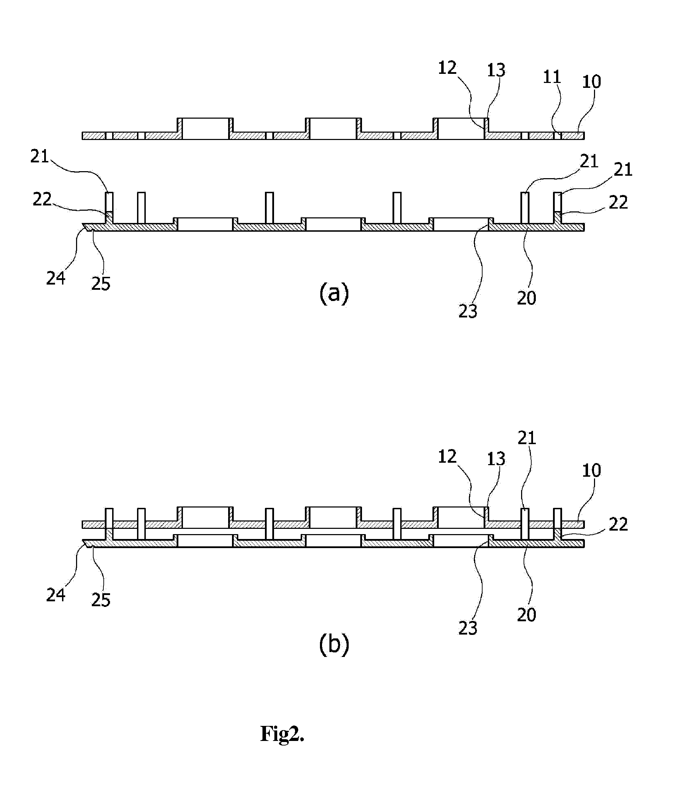

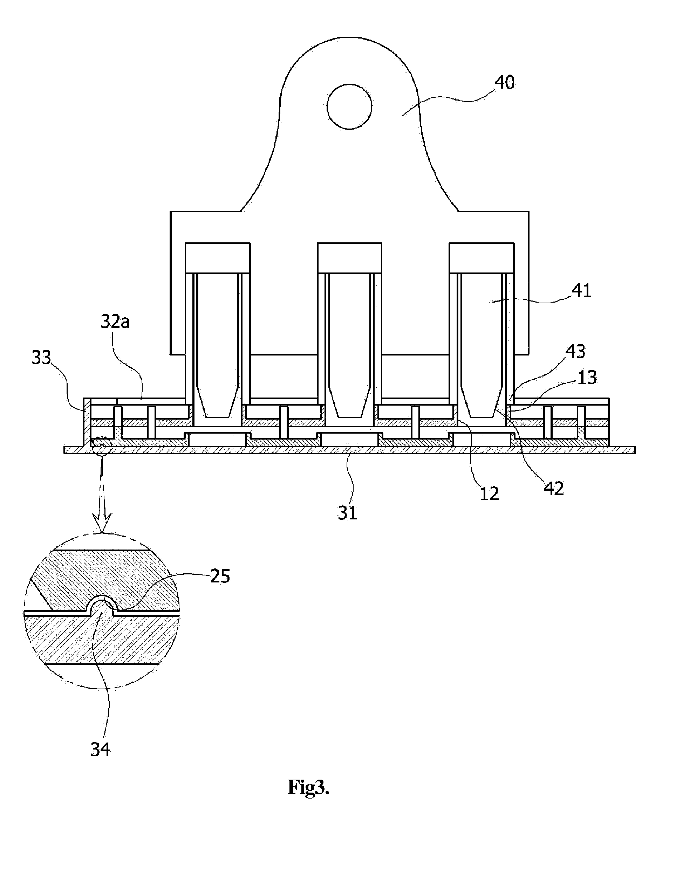

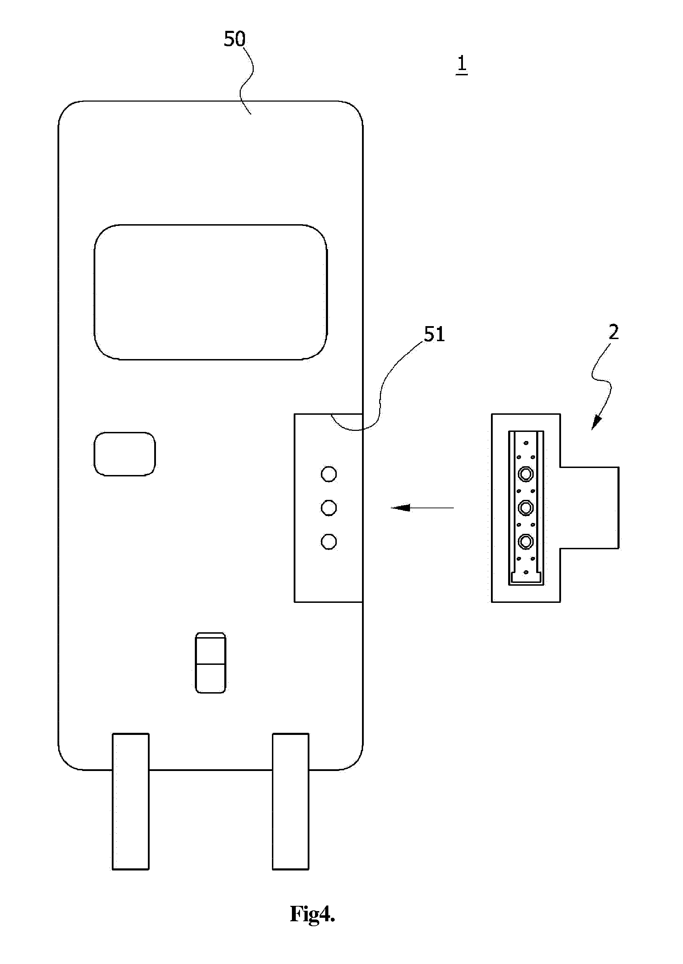

Fig. 1 is an exploded perspective view illustrating an optical-sensor strip according to an embodiment of the invention, Figs. 2A and 2B are cross-sectional views illustrating a support and a cover shown in Fig. 1, Fig. 3 is a cross-sectional view illustrating a state in which the support and the cover are inserted into a fixing frame, and Fig. 4 is a plan view illustrating a diagnostic apparatus according to an embodiment of the invention.

As illustrated in Figs. 1 to 4, a diagnostic apparatus 1 according to an example of the invention includes a body 50, an optical-sensor strip 2, and a multi-injector 40.

The optical-sensor strip 2 includes a support 20, a cover 10 that opens and closes one side of the support 20, a fixing frame 30 into which the support 20 and the cover 10 are inserted in a state in which the cover 10 covers the support 20. The diagnostic equipment has a body 50 with an insertion unit 51 through which the fixing frame 30 can be inserted in a widthwise direction (the perpendicular direction to the longitudinal direction of the cover and the support).

The support 20 has a plate shape and includes a plurality of insertion protrusions 21 on the surface thereof on which a sample pad (not shown), which stores a sample, is stacked. The cover 10 has a plate shape so as to cover a side of the support 20 and includes pairs of insertions holes 11 that are matched with the insertion protrusions 21 and into which the insertion protrusions 21 are respectively inserted. The cover 10 further includes a plurality of injection holes 12 that are formed to penetrate a body of the cover 10. The cover 10 still further includes injection protrusions 13 each protruding from the surface of the cover by a predetermined height and extending along the corresponding injection hole 12. The injection protrusion 13 protrudes such that it can come into contact with supporting protrusions 43 of the multi-injector 30 to be described below. The injection protrusion 13 may have a height that is set such that only an end portion of a cuvette 41 can be inserted into the injection hole 12 when the injection protrusion 13 is in contact with the supporting protrusion 43. The cover 10 and the support 20 may be molded products of a macromolecular polymer or may be formed of metallic sheet, rubber, or plastic. The supporting protrusions 43 may have a structure in which it can come into contact with the injection protrusion 13. Alternatively, the supporting protrusion 43 may have a structure in which the supporting protrusion 43 comes into direct contact with the top surface of the cover 10 depending on the length of the supporting protrusion 43.

The support 20 has through holes 23 that penetrate the body of the support 20 and overlap the injection holes 12. The support 20 further includes insertion protrusion 21 arranged at both sides of each through hole 23. It is preferable the insertion protrusions 21 are formed such that every two insertion protrusions 21 arranged in the direction of width of the support 20 makes a pair. At both end portions of the support 20 in the longitudinal direction, gap-forming protrusions 22 are respectively formed to extend in a direction perpendicular to the longitudinal direction of the support 20 and to have a predetermined height. The insertion protrusions 21 may be additionally provided at the centers of the gap-forming protrusions 22, respectively. For this case, the cover may be further provided with the insertion holes 11 corresponding to the additional insertion protrusions 21 formed at the centers of the gap-forming protrusions 22.. As illustrated in the drawing, a protrusion having a predetermined height may also be formed along the circumference of the through hole 23 as necessary. An end portion of the support 20 in the longitudinal direction has a slopped surface 24 so that the support 20 can be easily inserted into the fixing frame 30. The slopped surface 24 also serves to indicate a direction in which the support 20 is inserted. That is, when the user attempts to insert a combined structure of the cover 10 and the support 20, the user first checks the slopped surface 24 formed in the support 20 with eyes and then inserts the support 20 in the fixing frame 30 from the end portion of the support 20 which is formed of the slopped surface.

The fixing frame 30 includes a base plate 31 with a handle portion 35 formed on a side thereof, a guide portion 32 that includes wall portions protruding from the surface of the base plate 31 by a height that corresponds to the sum of the height of the support 20 and the height of the cover 10 in a combined state and extending along both sides of the base plate 31, and projected portions 32a bent from the respective top ends of the wall portions toward the center line of the base plate 31. One of both open ends of the guide portion 32 in the longitudinal direction is provided with a barrier wall 33. Alternatively, the fixing frame 30 may not include the barrier wall 33 that closes one end of the guide portion 32. However, it is preferable that the fixing frame includes the barrier wall 33.

A stopping recess 25 is formed to be recessed in an end portion of the support 20 and a stopping protrusion 34 is formed at an end portion of the base plate 31 so as to be temporarily inserted into the stopping recess 25. When the support 20 is fully inserted into the guide portion 32, the stopping protrusion 34 is inserted into the stopping recess 24 so that the fixed state is temporarily maintained.

The support 20 is covered with the cover 10, and the top portion of the combined structure of the support 20 and the cover 10 is uniformly pressed by the projected portion 32a of the fixing frame 30. Accordingly, this structure can reduce a measurement error due to a compression irregularity that has occurred in a related art when the support 20 is cover 10 is combined.

The gap-forming protrusions 22 serve to maintain a uniform gap between the cover 10 and the support 20 so that a measurement error can be reduced. That is, irregular reaction speed and poor reproducibility of repetitive measurement values originating from a combined structure in which combining force is irregular can be compensated, and a likelihood of a measurement error due to the deformation of the strip can be reduced.

AS illustrated in Fig. 3, a multi-injector 40 is provided with a plurality of cuvettes 41 arranged in line. The cuvette 41 is a small vessel to store a liquid and is mainly used for optical measurement. A typical cuvette for use in photoelectric colorimetric analysis is made of Pyrex glass or quartz. It also may be made of plastic or ceramic.

A plurality of injection holes 12 is formed in the cover 10. A sample can be injected into the strip through the respective injection holes 12 using the multi-injector 40 so that different amounts of sample may be injected for each way of the cuvette, or a plurality of samples can be injected at a time. Each cuvette 41 is marked with guide lines so that the user can recognize the volume of the sample in the cuvette. Accordingly, each way may be supplied with a different amount of sample by using a pipette. The end portion of each of the cuvettes 41 that form the multi-injector 40 has an inclined surface 24 so that they can be easily inserted into the injection holes 12.

As illustrated in Fig. 3, the supporting protrusions 43 come into contact with the end portions of the injection protrusions 13 and the end portions of the cuvettes 41 are inserted into the injection holes 12.

As such, since the supporting protrusions 43 are supported by the injection protrusions 13, samples can be precisely and accurately injected without shaking when the samples are injected. For such a reason, dispending a sample can be exactly performed and an error due to the user's mistake can be reduced.

In the multi-injector 41 in which a plurality of cuvettes 41 are installed, supporting protrusions 43 protrude around the respective cuvettes 40 in the longitudinal direction of the cuvettes 41. It is preferable that the supporting protrusions 43 are formed along the cuvettes 40 such that the supporting protrusions 43 can come into contact with the ends of the injection protrusions 13.

Fig. 4 is a plan view illustrating a diagnostic apparatus according to an embodiment of the invention. As illustrated in Fig. 4, a body 50 of a diagnostic apparatus 1 has an insertion unit 51 so that a fixing frame 30, in which the combined cover 10 and support 20 is inserted, can be inserted in the widthwise direction into the body 50. The insertion unit 51 has a width corresponding to the length of the fixing frame 30 so that the fixing frame 30 can be inserted into the insertion unit 51 in the perpendicular direction to the longitudinal direction of the support 20. That is, the body 50 mounted with the optical-sensor strip having the structure described above is provided with the insertion unit 51 so that the fixing frame 30, in which the combined support 20 and cover 10 is installed, can be inserted into the insertion unit 51 in the perpendicular direction to the longitudinal direction of the support 20. In the insertion unit 51, an optical-sensor is positioned where the injection hole 12 is overlapped.

The diagnostic apparatus according to the invention is a complex diagnostic apparatus that can measure electrical and chemical components of a sample and can measure a plurality of measurement items at a time. Accordingly, it can be used as a sensor for determining GOP, GPT, GGT liver diseases as well as a sensor for determining the concentration of cholesterol. It also can be used to determine anemia.

That is, optical measurement can be made by the insertion unit formed at a side portion of the body 50, and electrical and chemical measurements can be made by an electrical and chemical measurement portion formed at an end portion of the body 50 and an electrical and chemical strip inserted into the electrical and chemical measurement portion.

For this case, the insertion unit 51 is structured such that the fixing frame 30, in which the cover 10 and the support 20 are inserted, can be inserted in the widthwise direction. Accordingly, it is possible to reduce an error due to the deformation or bending of the strip that has occurred in the related art when the strip is inserted in the longitudinal direction.

Although a preferred embodiment of the present invention has been described for illustrative purposes, those skilled in the art will appreciate that various modifications, additions and substitutions are possible, without departing from the scope and spirit of the invention as disclosed in the accompanying claims.

## Claims

1. An optical-sensor strip comprising:
a support 20 including a plate shape, the support 20 having a plurality of insertion protrusions 21 formed on an upper surface thereof on which a sample pad containing a sample is mounted,
a cover 10 placed on the upper surface of the support 20, the cover 10 including insertion holes 11 to engage with the insertion protrusions 21, and a plurality of injection holes 12 penetrating the cover 10; and
a fixing frame 30 including a guide portion 32 in which a combined structure of the support 20 and the cover 10 is inserted.

2. The optical-sensor strip according to claim 1, wherein the cover 10 is provided with injection protrusions 13 that protrude from a surface thereof along a circumference of the injection holes 12.

3. The optical-sensor strip according to claim 2,
wherein the support 20 includes through holes 23 that are formed to penetrate a body of the support 20 at positions corresponding to the injection holes 12, and
wherein at least two insertion protrusions 21 are symmetrically arranged at around the through hole 23.

4. The optical-sensor strip according to claim 1,
wherein the support 20 includes gap-forming protrusions 22 at both end portions thereof for maintaining a gap between the cover 10 and the support 20.

5. The optical-sensor strip according to claim 4,
wherein at a center portion of each of the gap-forming protrusion 22, an additional insertion protrusion 21 is provided.

6. The optical-sensor strip according to claim 1,
wherein the fixing frame 30 includes a base plate 31 having a handle portion at a side thereof; and
wherein the guide portion 32 has wall portions protruding from a surface of the base plate 31 to extend along both sides of the combined structure of the support 20 and the cover 10, and projected portions 32a bent from end of each of the wall portions in a direction approaching each other.

7. The optical-sensor strip according to claim 6,
wherein the fixing frame has a barrier wall that closes an open end of the guide portion.

8. The optical-sensor strip according to claim 6, wherein a stopping recess 25 is formed on an end portion of a lower surface of the support 20 and a stopping protrusion 34 is formed on the base plate 31 so that the stopping protrusion 34 is temporarily received in the stopping recess 25 when the support 20 is fully inserted in the guide portion 32.

9. The optical-sensor strip according to claim 8,
wherein an end portion of the support 20 has a slopped surface 24.

10. A diagnostic apparatus comprising:
the optical-sensor strip according to claims 1 or 6;
a body 50 having an insertion unit 51 in which the optical-sensor strip is inserted in a widthwise direction of the optical-sensor strip; and
a multi-injector 40 including a plurality of cuvettes 41 with end portions inserted in the injection holes 12, and supporting protrusions 43 that are protruded in a longitudinal direction of the cuvettes 41 ,
wherein ends of the supporting protrusions 43 are in contact with the cover 10 so that a state in which the end portions of the cuvettes 41 are inserted in the injection holes 12 is remained.
